# EUROPEAN PATENT APPLICATION

(11) **EP 4 480 397 A1**
(43) Date of publication of application: **25.12.2024**
(21) Application number: 23180977.3
(22) Date of filing: 22.06.2023
(51) Int. Cl.: A61B 5/01, A61B 5/028

(54) **BODY FLUID FLOW SENSING**

(71) Applicant: Koninklijke Philips N.V., 5656 AG Eindhoven (NL)
(72) Inventor: JOHNSON, Mark Thomas, Eindhoven (NL); MENA BENITO, Maria Estrella, Eindhoven (NL); PAULUSSEN, Elvira Johanna Maria, Eindhoven (NL); PERRONE, Antonio Luigi, Eindhoven (NL)
(74) Representative: Philips Intellectual Property & Standards

(57) **Abstract**

A device, such as a personal care device, including means for applying a thermal stimulus (hot or cold) to a location or region on or beneath a tissue/skin surface, and thermal imaging means for acquiring thermal imagery of a region of the tissue spatially displaced from the location of application of the thermal stimulus. A signal trace of the applied thermal stimulus at the displaced imaging location is detected, for example as a function of time, and this information is used to determine body fluid flow information.

## Description

### FIELD OF THE INVENTION

The invention relates to the field of personal care devices and/or personal health devices.

### BACKGROUND OF THE INVENTION

Blood flow measurements are valuable for a wide range of physiological and clinical scenarios. Blood flow in humans is mostly distributed in a cycle from large vessels (arteries) to peripheral body areas and back to large vessels (veins). When the blood circulatory system functions normally, blood flows freely, transporting oxygen and nutrients to tissues and organs.

Peripheral circulation may decrease under pathological conditions, causing poor circulation. This can result in symptoms that may vary from tingling sensations in the extremities to varicose veins.

With regards to varicose veins, these are dilated, inflamed, tortuous and elongated veins affecting especially (though not exclusively) the superficial veins of the lower limbs. They can in some cases be visible from a surface of the skin, appearing as a patchwork of bulging blue and purple lines. Varicose veins sufferers may experience symptoms such as aches, swelling and itchiness.

Turbulent blood flow in varicose veins may lead to significant complications such as slow-healing ulcers, chronic venous ulcers and spontaneous bleeding. Local blood changes in varicose veins including increased thrombotic potential and inflammation may cause chronic venous insufficiency, thrombophlebitis and deep vein thrombosis.

Early detection of suspected varicose veins may help to prevent further progress and development of varicose veins.

Further to varicose veins, Raynaud's phenomenon is another disease that can affect a subject's blood vessels. This condition causes decreased blood flow to the fingers. This causes white discoloration of the fingers, followed by cyanosis and pain. In some cases, it may also cause less blood flow to the ears, toes, nipples, knees, or nose. This happens due to spasms of blood vessels in those areas. The spasms may happen in response to cold, stress, or emotional upset.

Another disease which can affect a subject's blood vessels is deep vein thrombosis (DVT). This is caused by a blood clot forming in one or more of the deep veins in the body, usually in the legs. Such blood clots can have a variety of causes, such as damage to a vascular inner lining, decreased blood flow rate (for example, from prolonged inactivity), and increased tendency of the blood to clot. Symptoms of DVT can include swelling in the affected leg, pain or tenderness in the leg, red or discolored skin on the leg, or a feeling of warmth in the affected leg. However, it is also possible for DVT to be initially symptomless.

In many cases, conditions such as varicose veins, Raynaud's phenomenon, and deep vein thrombosis may not present any obvious signs or symptoms, at least in their early stages.

In the current state of the art, diagnosis and monitoring of those conditions must be done by a healthcare professional. Objective diagnostic methods, such as color Doppler ultrasound, MR angiography, perfusion scintigraphy, plethysmography, laser speckle contrast imaging and thermography are currently used. Most of these require dedicated clinical expertise to perform the measurement or to interpret results.

Plethysmography relies on the measurement of bulk changes in limb dimensions caused by changes in blood volume, thereby only providing an estimate of flow to the entire limb. Measurements typically involve strain gauges wrapped around the limb to quantify dimensional changes or, in the case of photoplethysmography, optical illumination to identify changes in optical absorption, both of which follow from changes in blood volume.

Ultrasound techniques rely on measuring acoustic Doppler shifts. Similar Doppler shifts in optical signals form the basis for laser measurements. Laser Speckle Contrast Imaging (LSCI) can also be used to measure blood flow. This is based on a phenomenon wherein, when coherent light, such as that from a laser, hits a surface, it creates a "speckle" pattern of light and dark regions due to interference. If the surface is moving (as in fluid flow), the speckle pattern changes over time. By analyzing these changes, it is possible to measure the movement of the surface.

The above methods for blood flow each have the disadvantage of requiring expert clinical administration or interpretation due to their complexity. Some are furthermore invasive.

It is also known to employ thermal methods for estimating blood flow. These utilize flow-induced thermal transport and/or thermodilution to provide an indication of blood flow. One set of known methods comprises intravascular injection of a known volume of fluid having a known temperature (being different to blood temperature) into the bloodstream and recording the corresponding downstream temperature changes. A cold fluid is often used as an indicator in the thermodilution method, since there is less risk of harm than in the case of using a hot fluid.

Other thermal approaches make use of metal heating and sensing plates applied to the skin. Here, blood flow in the tissue influences the time and/or spatial dependence of the thermal response, which provides a means to determine spatial variations in effective thermal conductivity. From this, information about regional perfusion can be inferred.

The known thermal methods also require expert clinical administration. For example, the fluid-based method is invasive, and the plate-based method requires careful execution and expert analysis of results.

Aside from blood flow, measurement of the flow of other body fluids may also be of value, for example breast milk, or lymph fluid.

Simpler methods for estimating body fluid flow information would be of value.

### SUMMARY OF THE INVENTION

The invention is defined by the claims.

According to examples in accordance with an aspect of the invention, there is provided a device for application in use to a tissue surface area, comprising: a thermal stimulus generator for applying a thermal stimulus to one or more locations of the tissue on or beneath the tissue surface area; a thermal imaging subsystem including at least one thermal imaging camera, for thermally imaging a region of the tissue surface area; and a controller. The controller is adapted to: receive thermal imaging data acquired by the thermal imaging subsystem during or subsequent to application of the thermal stimulus; extract a thermal signal from the thermal imaging data (e.g. corresponding to a trace/signal of the applied thermal stimulus in the tissue); and determine body fluid flow information based on the extracted thermal signal.

Embodiments of the invention are based on the principle of measuring a thermal trace of an applied thermal stimulus at a location of the tissue displaced from a location of applying the thermal stimulus. The thermal stimulus heats a region of the body fluid, and this region is then fluidly transported along the direction of fluid flow. The device is arranged for detecting this travelling thermal pulse carried by the motion of the fluid. This may be referred to as translational thermal transport by body fluid flow.

In some embodiments, the thermal stimulus generator is adapted to generate one or more thermal stimulus pulses.

In some embodiments, the thermal signal which is extracted from the thermal imaging data may be a thermal pulse wave signal.

In some embodiments, extracting the thermal signal comprises extracting a thermal pulse wave having a peak or center point which is displaced, e.g. laterally, from a location of application of the thermal stimulus. Laterally may mean along a direction parallel with a surface of the tissue.

In some embodiments, the thermal pulse wave to be detected is a Gaussian pulse wave.

In some embodiments, the extracting the thermal signal comprises detecting in the thermal imaging data a thermal pulse wave of a pre-defined shape or mathematical form. e.g. a Gaussian.

In some embodiments, the thermal imaging data comprises a time series of thermal image frames spanning an imaging period.

In some embodiments, the extracting the thermal signal comprises detecting presence and/or location of a thermal pulse wave in each of at least a subset of the time series of thermal image frames. This may thereby obtain a thermal pulse wave signal comprising a sample of the thermal pulse wave at each of a series of sampling time points.

The controller may be adapted to determine at least one property of a travel path of the thermal pulse wave signal across an imaging field of view of the thermal imaging subsystem based on plotting a location of a fiducial point of the thermal pulse wave (e.g. a location of a peak or center point of the thermal pulse wave) at each of a series of sampling time points (e.g. in each of a series of acquired thermal image frames).

In some embodiments, the thermal imaging data comprises a time series of thermal image frames spanning an imaging period, and wherein the controller is adapted to identify a temporally first frame of the series of frames in which the thermal pulse wave is present/detectable. If a distance, across a direction parallel with the tissue surface, between the point of application of the thermal signal and the boundary of the image frame is known, then the time point corresponding to this first frame can be used to compute an indication of thermal transport speed by the fluid flow, and thus a parameter indicative of flow speed (e.g. at least correlated with flow speed).

In other words, if a boundary of the imaged region is a known distance from the application point of the thermal stimulus, then a time of capture of the first frame can be used to determine a speed of travel of the pulse, and consequently infer fluid flow information.

The point of application of the thermal stimulus may be displaced, across a direction parallel with the tissue surface, from a central point of the imaging field of view of the at least one thermal imaging camera by a distance of at least 1cm, for example at least 1.5cm, for example at least 2cm. This may be achieved by providing a housing or support frame which holds the thermal stimulus generator and camera a fixed distance from one another of e.g. at least 1cm, or at least 1.5cm or at least 2cm, or may be achieved through implementing a sequential measurement approach in which the device is moved position between stimulus generation and imaging. The latter option will be described in greater detail later.

In some embodiments, the measured flow information may relate to superficial/peripheral blood flow or deep vein/artery blood flow. Alternatively, it may related to another flowing bodily fluid such as milk or lymph fluid.

In some embodiments, the controller is adapted to record extracted thermal signal data and/or body fluid flow information from multiple measurement sessions in a memory and is adapted to determine longitudinal body fluid flow information based thereon. This may for example be trend information indicative of a trend in body fluid flow rate at a location of the body over time.

By way of example, the controller may be adapted to compare a value of a body fluid flow parameter measured at a later time point with a value of a body fluid flow parameter measured at an earlier time point to determine the longitudinal body fluid flow information.

In some embodiments, the longitudinal body fluid flow information comprises an indication of a longitudinal trend in a value of a body fluid flow parameter over time. The controller may be adapted to apply a pre-defined diagnostic algorithm to the longitudinal trend, the diagnostic algorithm adapted to generate a diagnostic classification based on the input longitudinal blood flow trend.

As mentioned above, in some embodiments, the thermal stimulus generator may be adapted to generate one or more thermal stimulus pulses. This may be used to create a localized heat or cool pulse in a vein or artery at the location to be measured.

In preferred embodiments, the thermal imaging camera is, during capture of the thermal imagery, at a location substantially thermally uninfluenced by thermal diffusion of the thermal stimulus.

In some embodiments, device is structured such that, when the device is applied to the tissue surface for measurement, the thermal stimulus generator is applied thermally coupled with the tissue surface, and the thermal imaging camera is supported at a position not touching the tissue surface, for example suspended above the tissue surface. The thermal stimulus generator is for example touching the tissue surface.

In some embodiments, the device comprises a tissue-facing area, and wherein the thermal stimulus generator and at least one thermal imaging camera of the thermal imaging subsystem are supported spaced from one another at the tissue facing area, for interaction with the tissue in use at tissue locations spaced from one another. For example, they may be spaced from one another by at least 1cm, for example by at least 1.5cm, for example by at least 2cm. In other words, in some embodiments, the thermal imaging subsystem acquires a thermal image a distance away from the temperature stimulus.

In some embodiments, the at least one thermal imaging camera is spaced from the thermal stimulus generator by a distance, x, and wherein a body fluid flow parameter is determined based on: a time delay between generation of a thermal stimulus by the thermal stimulus generator, and first detection of a resulting thermal pulse wave signal in an imaging field of view of the at least one thermal imaging camera.

The body fluid flow parameter may be determined based on a ratio of the distance, x, to the time delay.

In some embodiments, the device is arranged such that when applied in use to the tissue surface area, the thermal imaging subsystem is arranged for acquiring first thermal imaging data of at least a first tissue region and second thermal imaging data of a second tissue region, the first tissue region being spaced from the thermal stimulus generator in a first direction across the tissue surface and the second tissue region spaced from the thermal stimulus generator in a second direction across the tissue surface, opposite to the first direction.

This allows for acquiring thermal measurements both upstream and downstream (relative to flow of the body fluid). The downstream signal may contain a component both of translational thermal transport by motion of the fluid and of thermal diffusion. The upstream signal solely contains a thermal diffusion component. By subtracting the upstream signal (or an inversion of it) from the downstream signal, the thermal diffusivity component can be removed from the measurement, leaving solely the component related to blood flow.

To implement this approach, in some embodiments, the thermal imaging subsystem comprises at least a first thermal imaging camera for acquiring the first thermal imaging data and a second thermal imaging camera for acquiring the second thermal imaging data. The first thermal imaging camera may be spaced from the thermal stimulus generator in the first direction across the tissue-facing area of the device, and the second thermal imaging camera may be spaced from the thermal stimulus in an opposite direction across a tissue facing area of the device.

The controller may be adapted to detect a thermal pulse wave signal in one of the first and second thermal imaging data. The controller may be further adapted to measure a background thermal diffusion signal in the other of the first and second thermal imaging data. The controller may be further adapted to estimate a background thermal diffusion signal present in the one of the first and second thermal imaging data having the thermal pulse wave signal based on the measured background thermal diffusion signal and subtract the estimated background diffusion signal from the detected thermal pulse wave signal to obtain a corrected thermal pulse wave signal. The controller may be further adapted to determine the body fluid flow information based on the corrected thermal pulse wave signal.

Estimating the background thermal diffusion signal may comprise: computing a spatial inversion or reflection of the measured background signal about an axis aligned laterally with a location of application of the thermal stimulus. If the imaging fields of view of the two cameras are positioned symmetrically about the location of the thermal stimulus generator, then the inversion or reflection will yield the corresponding signal in the other of the two thermal image regions. The inverted background diffusion signal can then be subtracted from the detected thermal pulse wave signal to obtain the corrected thermal pulse wave signal.

In some embodiments, the controller is adapted to control acquisition of the thermal imaging data to start simultaneously with activation of the thermal stimulus.

In further embodiments, the controller is adapted to implement a sequential measurement process, for application of a thermal stimulus with the device positioned at a first location on the tissue, and acquisition of thermal imaging with the device positioned at a second location on the tissue, spaced from the first location, and the measurement process comprising: controlling activation of the thermal stimulus; controlling deactivation of the thermal stimulus; controlling acquisition of the thermal imaging data subsequent to deactivation of the thermal stimulus, optionally after a pre-defined pause period. The user may move the device form the first location to the second location.

In some embodiments, the device may include a sensory output element, e.g. including one or more visual output elements or one or more acoustic output elements, and wherein the controller is adapted to generate a sensory output upon deactivation of the thermal stimulus for prompting a user to move the device to the second location.

In some embodiments, the controller may be adapted to implement a calibration procedure comprising: controlling the thermal stimulus generator to incrementally sweep through an increasing sequence of thermal stimulus intensity levels, starting from a lowest intensity level; at each intensity level: acquiring thermal imaging data, extracting a thermal signal from the thermal imaging data, and determining a signal strength of the thermal signal; identifying a lowest thermal stimulus level at which the signal strength of the thermal signal meets a pre-defined threshold; and recording the identified lowest thermal stimulus level for use in subsequent measurement. In this way, the intensity of the thermal stimulus can be configured to the minimum possible to achieve good signal strength. This optimizes user comfort.

In some examples, the strength of the signal may be determined based on a magnitude of a peak of a thermal pulse wave signal.

In some embodiments, the aforementioned calibration procedure may comprise terminating the thermal stimulus sweep once a thermal stimulus intensity level has been identified for which the corresponding thermal signal meets the pre-defined threshold. This minimizes the duration of the calibration procedure.

In some embodiments, the device may further include localization means for detecting a location of the device on the body. The controller may be adapted to implement a device navigation phase, comprising: monitoring a detected location of the device and controlling generation of user output information for guiding a user to a pre-defined reference location. For example, the output information may take the form of one or more sensory outputs generated responsive to detection of a pre-defined reference location. This allows for measurements to be obtained at a same location on the body each time which is important for computing longitudinal trends.

With regards to the thermal stimulus generator, this can be adapted to generate a heat stimulus and/or a cold stimulus. In some embodiments, the thermal stimulus generator may comprise any one or more of a Peltier cooling element; a resistive heating element; a radio frequency heating element; a near infrared heating element; a High intensity Focused Ultrasound heating element; an infrared illumination source.

The proposed device may be used for inferring flow information about any of a variety of body fluids. One particularly advantageous application is for detecting blood flow. Thus here, the body fluid is blood, and wherein the body fluid flow information is blood flow information.

In one advantageous set of embodiments, the device may be a personal care device. The device thus may comprise a set of operative components for performing a personal care function.

By way of a non-limiting set of examples, the personal care device may be an optical hair removal device, e.g. an Intense Pulsed Light (IPL) epilator device; a shaver device; a breast pump; or an oral healthcare device, such as an oral cleaning device, e.g. a toothbrush.

Another aspect of the invention is a method for detecting body fluid flow information. The method comprises applying a thermal stimulus to one or more locations of a tissue surface area; using a thermal imaging subsystem including at least one thermal imaging camera to thermally image a region of the tissue surface area during or subsequent to application of the thermal stimulus; extracting a thermal signal from the thermal imaging data (e.g. corresponding to a signal/trace of the applied thermal stimulus); and determining body fluid flow information based on the extracted thermal signal.

The thermal imaging step may comprise thermally imaging a region of the tissue surface area which is spatially displaced from the location to which the thermal stimulus was applied, e.g. displaced by a distance of at least 1cm, e.g. at least 1.5 cm, e.g. at least 2 cm.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example device in accordance with one or more embodiments of the invention;
Fig. 3 schematically illustrates an example device in accordance with one or more embodiments;
Fig. 4 schematically illustrates application of an example device in use in accordance with one or more embodiments;
Fig. 5 schematically illustrates measurement of a thermal signal in accordance with one or more embodiments of the device; and
Fig. 6 illustrates an example device in accordance with one or more embodiments comprising thermal imaging means on either side of the thermal stimulus generator.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device, such as a personal care device, which includes means for applying a thermal stimulus (hot or cold) to a location or region or beneath a tissue/skin surface, and thermal imaging means for acquiring thermal imagery of a region of the tissue spatially displaced from the location of application of the thermal stimulus. A signal trace of the applied thermal stimulus at the displaced imaging location is detected, for example as a function of time, and this information is used to determine body fluid flow information.

The measurement approach is based on the principle that a subcutaneous body fluid flow path will translationally carry an applied thermal pulse along a travel path at a rate which correlates with a rate of the body fluid flow. Thus, detecting the signal of the thermal stimulus at a downstream location (relative to the flow direction) of the applied stimulus can yield information about flow, such as flow rate, or spatial patterning of flow.

Embodiments of the invention provide a non-invasive approach for monitoring of blood flow (or another flowing bodily fluid such as milk or lymph fluid) and detection of possible blood flow anomalies using a device (e.g. a personal health care device) provided with a thermal imaging component.

It is thus proposed to use a temperature stimulus (heating or cooling source) provided locally on or below the surface of subject's body location together with the use of a thermal camera to measure an indication of fluid flow (e.g. blood flow) and as such to detect possible fluid flow (e.g. blood flow) anomalies.

The temperature stimuli causes a change in temperature of the blood (or other body fluid), which then flows away from the location of the temperature stimulus.

The thermal camera may be positioned to image at a location displaced from the source of local heating or cooling, where the influence of thermal conductivity or diffusivity becomes minimal. As such, the mechanism differs from known methods in the art, which rely upon measurements very close to the heating source where thermal conductivity dominates. Furthermore, the use of a thermal camera to measure a thermal signal allows for measuring a spatially distributed thermal signal, e.g. a thermal pulse wave of a pre-defined characteristic shape or mathematical form, e.g. detection of a 2D or 3D Gaussian pulse wave or similar. This allows for specifically detecting a signal associated with translational thermal transport by fluid flow as opposed to direct thermal conduction or thermal diffusion. Thus is because thermal diffusion or conduction results in a thermal distribution centered at the point of application of the thermal stimulus and spread out in all direction around that point. This is different to a fluidly transported thermal signal which results in a thermal pulse having a center or peak point which, positionally, is displaced from the point of application of the thermal stimulus at a rate correlated with the rate of fluid flow.

Thus, more generally, it may be said that at least one set of embodiments of the invention are based on detecting a thermal pulse wave having a peak point or center point which, positionally, is displaced in a direction parallel with the tissue surface, from the point of application of the thermal stimulus. The use of a camera with a 2D field of view enables identification of such a pulse wave, as distinguished from a signal component associated with thermal diffusion, since at least a 2D spatial profile of thermal signals in the thermal imaging frame can be analyzed.

Optionally, both the heating or cooling source and thermal camera are integrated into a personal health care device. This advantageously enables the

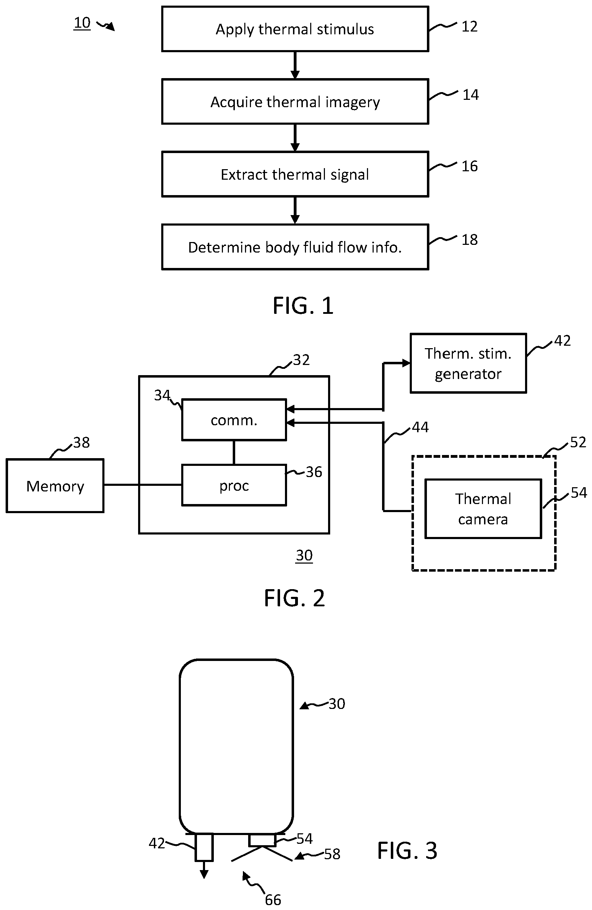

Fig. 1 outlines in block diagram form steps of an example method according to one or more embodiments. The steps will be recited in summary, before being explained further in the form of example embodiments.

The provided method 10 is for detecting body fluid flow information.

The method comprises applying 12 a thermal stimulus to one or more locations of a tissue surface area. This may be a heat stimulus or cool stimulus. This means generally a stimulus which induces heating or cooling of a region at or beneath the tissue surface. Ideally, the thermal stimulus is applied at least to a region of body fluid (e.g. blood) in a body fluid conduit, e.g. a blood vessel.

The method further comprises using a thermal imaging subsystem including at least one thermal imaging camera to thermally image 14 a region of the tissue surface area during or subsequent to application of the thermal stimulus. This preferably at least includes a region spatially displaced from the point of application of the thermal stimulus.

The method further comprises extracting 16 a thermal signal from the thermal imaging data. The aim is to detect a thermal signal which corresponds to a signal/trace of the applied thermal stimulus.

The method further comprises determining 18 body fluid flow information based on the extracted thermal signal. For example the body fluid information may comprise a parameter indicative of or correlated with fluid flow rate.

It is noted that, for the purposes of this document, fluid 'flow' refers to volume per unit time. Flow rate can be understood as synonymous as fluid flow.

The invention can also be embodied in hardware form, for example in the form of a processing device which is configured to carry out a method in accordance with any example or embodiment described in this document, or in accordance with any claim of this application.

To further aid understanding, Fig. 2 presents a schematic representation of an example controller 32 configured to execute a method in accordance with one or more embodiments of the invention. The controller is shown in the context of a device 30 which comprises the controller. The device is a device for application in use to a tissue surface area. The device may be a personal care device comprising a set of operational components for performing a personal care function.

By way of just one set of examples, the device may be any of: an optical hair removal device, e.g. an Intense Pulse Light (IPL) epilator device; a shaver device; a breast pump; or an oral healthcare device, such as an oral cleaning device, e.g. a toothbrush.

Fig. 2 shows only a schematic block diagram of components of the device.

The controller 32 alone represents an aspect of the invention. The device 30 is another aspect of the invention. The provided device need not comprise all the illustrated hardware components; it may comprise only subset of them.

The controller 32 may comprise one or more processors 36 configured to perform a method in accordance with that outlined above, or in accordance with any embodiment described in this document or any claim of this application. In the illustrated example, the controller further comprises an input/output 34 or communication interface.

In the illustrated example of Fig. 2, the device 30 further comprises a thermal stimulus generator 42 for applying a thermal stimulus to one or more locations of the tissue surface area. By way of example, the thermal stimulus generator may comprise or provide a thermal source for creating a thermal stimulus applied to tissue. By way of example, the thermal stimulus may take the form of a localized heat or cool pulse in a vein or artery.

By way of a non-limiting set of examples, the thermal stimulus generator may comprise any one or more of a Peltier cooling element; a resistive heating element; a radio frequency heating element; a near infrared heating element; a High intensity Focused Ultrasound heating element (to create heat at a deep level in the skin); and an optical illumination source, e.g. an infrared or near infrared illumination source.

The thermal stimulus generator 42 may be adapted to apply or induce a thermal stimulus either at a surface of the tissue (e.g. application of a heat or cold source to the tissue surface), or at a location beneath the surface of the tissue, e.g. by high intensity light stimulus, e.g. infrared stimulation. For example, it is known the context of optical epilation to use high intensity pulses or infrared or near infrared light to stimulate heating beneath a surface of the tissue. In some embodiments, a similar technique could be used to generate a subcutaneous thermal stimulus. In some embodiments, the device may be a personal care device in the form of an Intense Pulsed Light (IPL) hair removal device which already includes an integrated optical source for applying IPL stimuli. This may be harnessed for a secondary function of fluid flow measurement.

In the illustrated example of Fig. 2, the device 30 further comprises a thermal imaging subsystem 52 including at least one thermal imaging camera 54, for thermally imaging a region of the tissue surface area. The at least one thermal camera is thereby used to detect temperature changes in the flowing fluid, e.g. blood (increased heat or cooling) as the fluid flows.

Preferably the field of view of the thermal imaging camera is situated beyond the extent of the applied thermal pulse, i.e. at a location substantially thermally uninfluenced by thermal diffusion from the initial thermal stimulus at the time of application, or within a certain time window thereafter, e.g. 1-2 seconds. This helps to ensure that the measured thermal response at the thermally imaged tissue region corresponds to thermal transport by fluid flow rather than by direct conduction from the thermal stimulus via the tissue.

Means for implementing a thermal camera are known. A thermal imaging camera, also known as an infrared camera or thermographic camera, is a device that forms an image using infrared radiation. It detects heat patterns or temperature changes in an imaged area. All objects with a temperature above absolute zero emit infrared radiation due to the thermal movement of particles. The amount of radiation increases with temperature. A thermal camera is adapted to detect this radiation and convert it into an image. The basic components of a thermal imaging camera are: an infrared sensor array, an optical system, and a signal processing unit. The infrared sensor array is adapted to detect infrared radiation. The sensor pixels convert incoming infrared energy into an electrical signal. The optical system is positioned in front of the sensor array and is adapted to focus the incoming infrared radiation onto the sensor array. Lenses used in thermal cameras are made from materials transparent to infrared radiation. The electrical signal from the sensor array may be sent to a signal processing unit which amplifies the signal and optionally convert it to a format that can be displayed.

Recent advances in technology have made it possible to miniaturize thermal imaging cameras significantly. For example, microbolometer technology allows the creation of small, uncooled infrared detectors which are suitable for use in handheld devices. These compact thermal cameras can be integrated into a small handheld device.

The device 30 may further comprise a memory 38 for storing computer program code (i.e. computer-executable code) which is configured for causing the one or more processors 36 of the controller 32 to perform the method as outlined above, or in accordance with any embodiment described in this disclosure, or in accordance with any claim. The memory may additionally or alternatively be used to store longitudinal blood flow information. For example, the controller 32 may be adapted in some embodiments to record extracted thermal signal data and/or body fluid flow information from multiple measurement sessions in a memory and may be adapted to determine longitudinal body fluid flow information based thereon.

The invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

Advantages of the proposed device include the following. The measurement approach is non-invasive. The method makes use of a "moving heat front" versus the `locally stationary heat spot" approach used currently in thermal methods. The method is appropriate for use in home-care devices operated by a novice user.

Fig. 3 schematically illustrates an outline of an example device 30 in accordance with one or more embodiments. The device comprises a tissue facing area 66 which is adapted to face a tissue surface area during use. At least part of the tissue facing area may contact the tissue surface. As illustrated, the thermal stimulus generator 42 and the at least one thermal imaging camera 54 are arranged disposed at the tissue facing area 66. For example, a thermal output area of the thermal stimulus generator 42 may be exposed at the tissue facing area, for facing tissue during use, and an imaging aperture of the thermal camera may be arranged exposed at the tissue facing area for facing tissue during use. By way of example, the thermal stimulus generator 42 and/or the thermal imaging camera 54 may be mounted to or carried by a surface of the tissue facing area 66. The tissue facing area may be covered by an optically transmissive cover member (not shown), such that a surface of the cover member may be applied onto the surface of the tissue during use.

In the illustrated example the tissue facing area 66 is formed by a head portion of the device 30. The device comprises for example a housing which houses one or more operational components of the device.

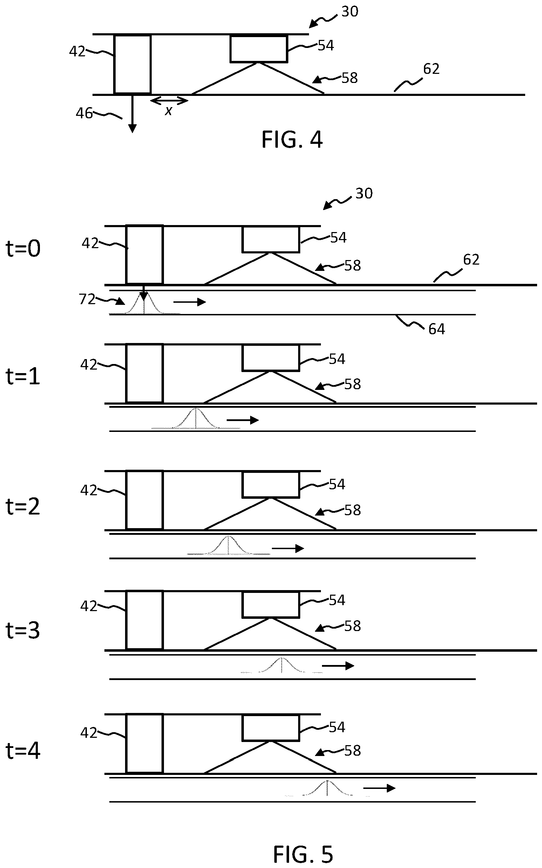

Fig. 4 schematically illustrates operation of the device 30 during use for applying a thermal stimulus and measuring a response.

Fig. 4 shows a lower portion of the device 30 engaged with a surface area of a tissue 62 which is to be stimulated and measured. By way of example, the illustrated section of the device might be a head portion of a handheld device, such as a personal care or personal health device.

In the illustrated example, the device 30 is structured such that, when the device is applied to the tissue surface 62 for measurement, the thermal stimulus generator 42 is applied thermally coupled with the tissue surface 62, and at least one thermal imaging camera 54 of the thermal imaging subsystem is supported at a position for imaging a region of the tissue surface. In the illustrated example, the at least one thermal imaging camera may be arranged not touching the tissue surface, e.g. suspended above the tissue surface, such that an imaging field of view 58 of the camera may span a region of the tissue surface.

In the illustrated example, the thermal stimulus generator 42 is arranged touching the tissue surface 62. For example, in a simple example, the thermal stimulus generator may apply the thermal stimulus 46 to the tissue by heat conduction. For example, the thermal stimulus generator comprises a heating element or cooling element, and contact heating or contact cooling of the tissue is performed via contact with the tissue surface. In other examples, heating or cooling might be performed of a region beneath a surface of the tissue through propagation of an energy wave, for example an optical wave or an acoustic wave. For example, heating beneath the surface of the tissue can be achieved using pulsed light in the manner discussed already above.

In the illustrated example, the thermal stimulus generator 42 and at least one thermal imaging camera 54 of the thermal imaging subsystem are supported spaced from one another (in a direction parallel with a surface of the tissue) at a tissue facing area of the device, for interaction with the tissue in use at tissue locations spaced from one another. As illustrated, the thermal stimulus generator 42 is spaced from the thermal imaging camera 54 by a distance x. More specifically, a boundary of a projection of the imaging field of view 58 of the thermal imaging camera on the incident tissue surface 62 is spaced from a boundary of the thermal stimulus generator 46 by a known distance, x. By tracking a time taken for a center or peak of a heat pulse to travel from the thermal stimulus generator to inside the field of view 58 of the thermal camera, a flow rate of a body fluid beneath the tissue surface can be determined.

Details regarding the measurement of flow information will now be discussed in more detail.

The general principle of measurement is to apply a temperature stimulus to a tissue area which contains a body fluid vessel, conduit or flow area and then to measure changes in temperature of the tissue at a location which is downstream from the point of temperature stimulation along a direction of flow of the fluid. Information related to the blood flow rate is extracted by relating the distance between the stimulus source (heat or cold) to the time that it takes to blood to change temperature (increase in the case of heat, decrease in the case of cold stimulus).

Fig. 5 illustrates schematically an example measurement process using a device in accordance with one or more embodiments.

The device 30 is applied to a tissue surface 62. The thermal stimulus generator 42 is controlled by the controller 32 (not shown in Fig. 5) to generate one or more thermal stimulus pulses. For example, this may comprise applying a heating stimulus for a defined time period. Fig. 5 shows the application of thermal stimulus 46 pulse at time t=0.

The application of the thermal stimulus results in creation of a detectable thermal signal in the fluid of a fluid-carrying conduit 64 (e.g. a blood vessel) underlying the tissue surface 62 location to which the device was applied. This thermal signal takes the form of a heated region in the fluid of the fluid conduit 64. Application of a thermal pulse to a localized stimulation area typically may result in a heat signal which has the form of a 2D or 3D Gaussian thermal wave. In other words, the temperature in the region of thermal stimulation as a function of position may have the form of a 2D or 3D Gaussian function. This temperature function response will be referred to a thermal signal.

If the thermal stimulus is applied to fluid which is flowing through the fluid conduit 64, then the heated region will be translationally transported along the conduit with fluid flow. In other words, the center of the thermal pulse signal will be transported along the conduit. Thus, in this case, the thermal signal in the fluid takes the form of a travelling pulse wave. This will be referred to as a pulse wave signal.

Fig. 5 shows a schematic representation of a travelling Gaussian pulse wave signal 72 which is carried along translationally with flow of fluid through the conduit 64. In addition to flow-induced transport of the pulse wave signal, the shape of the pulse will also diffusively spread out as a function of time. Thus, a center of the pulse wave moves translationally as a function of flow speed, and the shape of the pulse wave spreads out as a function of time. This spreading out is also schematically illustrated in Fig. 5.

As the thermal pulse wave signal 72 travels along the path of fluid flow, it will, at a certain time point, become detectable within the field of view 58 of the thermal imaging camera 54. This time point is schematically shown as t=2 in Fig. 5.

The controller is adapted to receive a time series of thermal image frames acquired by the thermal camera 54 following and/or during generation of the thermal stimulus 46. The time series of image frames spans an imaging period. Each thermal image is processed to detect and extract any thermal signal present in the frame. In some examples, this may comprise detecting presence of a thermal signal of a pre-defined mathematical form or shape, e.g. detection of a Gaussian pulse wave signal. The pre-defined shape or mathematical form may be a 2D and/or 3D shape or form.

Once the pulse wave signal 72 is detected in the field of view of the camera 54, it may be repeatedly sampled at each of a series of sampling time points, i.e. sampled in each acquired thermal image frame. Extracting the thermal signal in each thermal image frame may thus comprise detecting presence and/or location of the thermal pulse wave in the thermal image frame.

In some embodiments, the series of samples of the pulse wave signal over the measurement period may be used to determine a plot or track of a motion of the pulse wave signal across the imaging field of view 58. For example, an indication of a speed and or location of the pulse wave signal may be determined based on this. This information may be used to compute an indication of flow rate of the fluid.

More generally, the controller 32 may be adapted to determine at least one property of a travel path of the thermal pulse wave signal across the imaged region of the tissue based on detecting a location of the thermal pulse wave (e.g. a location of a peak of the thermal pulse wave) at each of a series of sampling time points (e.g. in each of a series of acquired thermal image frames).

In some embodiments, where the thermal imaging data comprises a time series of thermal image frames spanning an imaging period, the controller may be adapted to identify a temporally first frame of the series of frames in which the thermal pulse wave is present/detectable.

If a boundary of the imaged region is a known distance from the application point of the thermal stimulus, then a time of capture of the first frame can be used to determine a parameter indicative of a speed of travel of the pulse, and consequently infer fluid flow information.

For example, the at least one thermal imaging camera in the illustration of the Fig. 4 and Fig. 5 is spaced from the thermal stimulus by a distance, x. A body fluid flow parameter may be computed based on: a time delay between generation of a thermal pulse by the thermal stimulus generator, and first detection of a resulting thermal pulse wave signal in the imaging field of view of the at least one thermal imaging camera. For example, the body fluid flow parameter is determined based on a ratio of the distance, x, to the time delay. Due to thermal delays between application of the temperature stimulus and a temperature change in the fluid, e.g. blood, this may not give an absolute measure of flow rate, but provides an indicator which can be expected to be correlated with flow rate. This may allow for example for monitoring longitudinal relative changes in body fluid, e.g. blood, flow in an area of the body. That is, the method may in some embodiments make use of longitudinal measurements for the assessment body fluid flow patterns in an area of the body. For example, by trending blood flow measures over multiple measurement sessions, it can be determined whether blood flow patterns are remaining consistent or whether they are changing.

As a more general statement, the controller may be adapted to record extracted thermal signal data and/or body fluid flow information from multiple measurement sessions in a memory and may be adapted to determine longitudinal body fluid flow information based thereon.

For example, the controller may be adapted to compare a value of a body fluid flow parameter measured at a later time point with a value of the body fluid flow parameter measured at an earlier time point to determine the longitudinal body fluid flow information. In other words, a body fluid flow trend may be computed as = fluid flow measured (day x) - fluid flow measured (base line= day 1).

The aforementioned longitudinal body fluid flow information may comprise an indication of a longitudinal trend in a measure of body fluid flow rate over time in some embodiments. The controller may be adapted to apply a pre-defined diagnostic algorithm to the longitudinal trend, and adapted to generate a diagnostic classification based on input longitudinal blood flow change information. For example, the diagnostic algorithm may be adapted to generate a classification indicative of any one or more of: normal flow, varicose veins, Raynaud's phenomenon, or deep vein thrombosis.

By way of example, a simple diagnostic algorithm or module may be adapted to compare a measured change in blood flow rate over a defined time period against one or more thresholds, wherein the thresholds correspond to risk thresholds or diagnostic thresholds for one or more blood flow related pathologies, such as varicose veins, Raynaud's phenomenon, or deep vein thrombosis. A rate of longitudinal change in flow may be computed and the thresholds may relate to threshold rates of change associated with different possible pathologies.

Following the application of a temperature stimulus, transfer of heat spatially can be caused by two different mechanisms: thermal conduction and fluid motion. In accordance with some embodiments, a method is proposed that differentiates more effectively between these mechanisms, and potentially others, to more accurately evaluate blood flow.

In detail, in some embodiments, the thermal imaging subsystem is configured to utilize two thermal imaging cameras, one positioned in front of the local temperature stimulus and the other behind it, for differential measurements. Alternatively, a wide view thermal camera can be situated to capture images on both sides of the thermal pulse. The camera located ahead of the heat pulse is designed to detect only thermal conduction, while the one behind is additionally capable of capturing the temperature signature from the blood flow, after it has been subject to the temperature stimulus. Therefore, due to the transportation of the thermal pulse by the blood following the application of the thermal stimulus, the temperature recorded by the detector located behind is different (either higher or lower, depending on the type of thermal stimulus used) compared to that recorded by the front detector.

Thus, to state this more generally, the thermal imaging subsystem may be configured to obtain first thermal imaging data from at least a first tissue region and second thermal imaging data from a second tissue region. The first tissue region is distanced from the thermal stimulus generator 42 in a first direction across the tissue surface, while the second tissue region is situated at a distance from the thermal stimulus in a second direction across the tissue surface, opposite to the first direction.

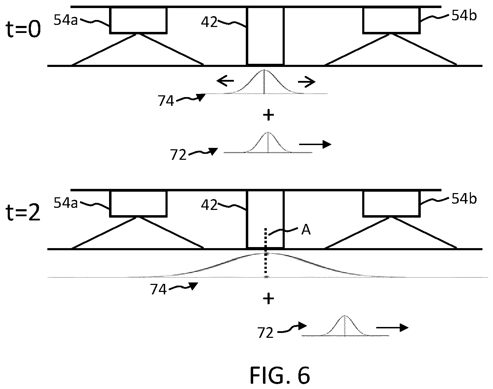

An example is illustrated schematically in Fig. 6. Here, the thermal imaging subsystem comprises at least a first thermal imaging camera 54a for acquiring the first thermal imaging data and a second thermal imaging camera 54b for acquiring the second thermal imaging data, and wherein the first thermal imaging camera is spaced from the thermal stimulus generator 42 in a first direction across the tissue-facing area of the device, and the second thermal imaging camera is spaced from the thermal stimulus in an opposite direction across a tissue facing area of the device.

As schematically illustrated in Fig. 6, the translational motion of fluid flow will transport a thermal pulse wave signal 72 along the direction of fluid flow. At the same time, heat diffusion across the tissue will introduce a background heat signal 74 indicative of the diffusive spreading of the heat pulse from its application point as a function of time. Thus the net thermal temperature at a given measurement point will be a sum of the travelling pulse wave signal 72 and the thermal diffusivity signal 74. Thus, it is proposed in some embodiments to subtract the background diffusive heat contribution 74 from the translational pulse wave contribution 72. This is accomplished by spatially flipping the spatial heat distribution captured by one the of the two cameras about an axis aligned with the location of the thermal stimulus generator 42 (i.e. the center point of the overall background thermal diffusion distribution) and then subtracting it from the spatial heat distribution recorded by the other of the cameras. This process yields a set of background-compensated thermal image data, from which the thermal signal can then be extracted.

In particular, the controller may be adapted to detect a thermal pulse wave signal 72 in either the first or second thermal imaging data and measure a background thermal diffusion signal 74 in the other set of data. For example, in the illustration of Fig. 6, the device is oriented such that the second camera 54b is arranged fluidly upstream from the thermal stimulus generator 42. Accordingly, the thermal pulse wave signal 76 is detected in the second thermal imaging data acquired by the second thermal camera. The first thermal imaging data acquired by the first camera 54a contains only a background thermal diffusion contribution. This measured background thermal diffusion signal in the first thermal imaging data of the first camera 54a can be spatially inverted or reflected about an axis (A) which is normal with the tissue surface to which the device is applied and which is aligned laterally with the center point of the thermal stimulus generator 42 (or more particularly the center point of the thermal stimulus generated by the thermal stimulus generator in the tissue). Since the first and second camera 54a have equal sized fields of view and are positioned laterally symmetrically about the thermal stimulus generator 42, then this inversion or reflection yields about axis A yields an estimate of the thermal diffusion contribution across the field of view of the second camera 54b. This can then be subtracted from the measured pulse wave signal 72 in the second thermal imaging data to yield a corrected thermal pulse wave signal. The controller may then determine body fluid flow information based on this corrected signal.

For implementation of the proposed sensing approach, the area imaged by the thermal imaging subsystem should include an area displaced from the location at which the thermal stimulus is applied, e.g. by a few cm. In some embodiments, this is achieved by providing a device comprising a housing or support structure which carries the thermal stimulus generator 42 and the at least one thermal imaging camera 54 at a defined distance from one another, such that, when the device is applied to the skin for use, the stimulus and the camera are spatially displaced from one another across the tissue surface. In this case, the controller can control acquisition of the thermal imaging data to start simultaneously with activation of the thermal stimulus for example.

However, according to a further set of embodiments, a sequential measurement approach can be considered, wherein the camera 54 and thermal stimulus generator 42 are disposed close to one another in/on the device, but wherein the user displaces the device relative to the tissue surface between generation of the thermal stimulus and capturing of the thermal imagery. Thus, use is made of the movement of the device itself to image at a position away from the stimulus.

In this case, a sequential mode of operation is preferred, whereby: in a first step the device is applied at a first device position and the thermal stimulus generator 42 controlled to generate the thermal stimulus; and in a second step, the device is moved to a second device position, and the camera captures thermal imagery at the second device position.

The controller may thus be adapted to implement a sequential measurement process, wherein a thermal stimulus is provided with the device positioned at a first location on the tissue, and thermal imaging is performed with the device positioned moved by a user to a second location on the tissue, spaced from the first location, and the measurement process comprising: controlling activation of the thermal stimulus; controlling deactivation of the thermal stimulus; and controlling acquisition of the thermal imaging data subsequent to deactivation of the thermal stimulus, optionally after a pre-defined pause period.

The apparatus may in some embodiments include a sensory output device, e.g. including one or more visual output elements or one or more acoustic output elements, and wherein the controller is adapted to generate a sensory output upon deactivation of the thermal stimulus for prompting a user to move the device to the second location.

For this approach to function optimally, it is necessary that the second device position is at a position where the fluid will flow. To increase the chance of this, it would be advantageous if the user could be advised in which direction to move the device. Furthermore, in order to provide an indication of flow velocity, also the distance moved by the device might be detected (using e.g. IMU or camera based approaches). There are various possible approaches to determine the direction to move the device. In some examples, the device could include an inertial measurement unit (IMU) which could detect the initial position and orientation of the device. With this information, the device could provide guidance (e.g., through a connected app or display or sensory output elements such as lights or sounds) to move the device in a specific direction. The controller may employ one or more machine learning algorithms trained to recognize patterns in thermal imaging data that suggest optimal directions for device movement. For example, it could recognize the gradients in a temperature field measured by the thermal camera before movement of the device, which are potentially associated with blood flow direction. Additionally or alternatively, the device could include sensors to measure skin elasticity or other physical properties, which might also suggest a preferred direction of movement.

Furthermore, in some embodiments, the controller may be adapted to further utilize additional information such as redness of skin and/or increased temperature variation in a measured tissue area in determining presence of any pathology of fluid flow. For example, the device could potentially incorporate a standard camera or specialized sensors to measure skin redness, which is typically associated with inflammation or increased blood flow. Image processing and/or machine learning algorithms could be used to quantify the degree of redness and potentially correlate this with particular physiological conditions or disease states. This information could then be combined with thermal data to provide a more comprehensive diagnostic assessment. With regards to temperature variation, this could refer to both spatial and temporal variation. Spatial variation may be measured as differences in temperature across different areas of the skin. This could help in identifying localized hot or cold spots that could indicate conditions like inflammation or poor circulation. Temporal variation may be measured as changes in temperature at a particular location over time. This could be useful in studying the heat dissipation pattern post the thermal stimulus, which could provide information about blood flow rates or metabolic activity.

In some embodiments, the level of thermal stimulus may be calibrated or normalized using a calibration procedure. Here, the controller may be adapted to initially sweep through a small-scale heat or cold stimuli range (temperature range).

For each temperature, a value of the body fluid flow parameter is calculated.

The temperature may then be systematically increased until a consistent value for the body fluid flow parameter is measured (e.g. signal to noise ratio meets a threshold level). This would help to prevent use of thermal stimuli which are larger than absolutely necessary.

To state this more generally, the controller may in some embodiments be adapted to implement a calibration procedure comprising: controlling the thermal stimulus generator to incrementally sweep through an increasing sequence of thermal stimulus intensity levels, starting from a lowest intensity level. At each intensity level, thermal imaging data is acquired and a thermal signal extracted from the thermal imaging data. A signal strength of each thermal signal is determined. A lowest thermal stimulus level at which the signal strength of the thermal signal meets a pre-defined threshold is identified. This identified lowest thermal stimulus level is then used in subsequent measurement. The strength of the signal may for example be determined based on a magnitude of a peak of a thermal pulse wave signal.

In some embodiments, the calibration procedure may comprise terminating the thermal stimulus sweep once a thermal stimulus intensity level has been identified which meets the pre-defined threshold. This reduces the duration of the calibration procedure.

It is preferable that the measurements of body fluid flow are performed at the same location on the body during each measurement session so that measurements over time are properly comparable.

In some embodiments therefore, the device may include device localization means for detecting a location of the device on the body. The controller is adapted to implement a device navigation phase, comprising monitoring a detected location of the device and controlling generation of a sensory output responsive to detection of a pre-defined reference location. For example, the device may store a record of a defined reference location. This might be set by a user in an initial device setup phase for example. Another form of user guidance may instead be generated based on the localization information.

With regards to the localization means, multiple options are possible. One approach may be the use of at least one inertial measurement unit (IMU) integrated in the device. An IMU is able to measure linear and angular motion, usually in the form of three-axis acceleration and three-axis gyroscope data. Using this information, the position of the device on the body could be inferred, especially if the initial position of the device was known. Thus, the localization means in this case may comprise an IMU integrated in the device in combination with a localization processor module adapted to infer a body location based on the signal data output from the IMU.

Another approach may be to leverage skin features, based on use of a machine learning model. Here, image data, either from the thermal imaging subsystem or from a separate optical imaging unit integrated the device could be processed by a trained machine learning algorithm to detect a body location to which the device is being applied.

This would involve training a machine learning model to recognize different parts of the body based on unique features of the skin at each location. These features might for example include hair density, skin texture, color, or even subdermal features like veins or muscle structure.

Such a model could be implemented using a combination of sensors. For example, visual sensors (e.g. a small camera or optical sensor), might be used to capture skin texture and color, while other sensors (e.g. capacitive or ultrasound sensors) could potentially be used to capture subdermal features.

The model might be trained for each individual user.

The detection of device location could also be used in the sequential measurement approach discussed previously. For example, the device could monitor a location of the device and alert the user once they have navigated correctly to a preferred second location for acquiring the thermal imaging data.

In some embodiments, the thermal stimulus generator may use infrared (IR) light to generate the thermal stimulus. Different wavelengths of IR light penetrate skin to different depths. This provides a means for using different wavelengths of light to differentiate between superficial and internal varicose veins based on their depth in the skin. Varicose veins are enlarged, swollen, and twisting veins, often appearing blue or dark purple. They most commonly occur in the legs and are caused by faulty valves in the veins. Some varicose veins are located close to the surface of the skin (superficial), while others are located deeper (internal).

Different wavelengths of infrared light penetrate different distances into the skin. Shorter wavelengths penetrate less deeply than longer wavelengths. By acquiring flow measurements at each of a range of wavelengths for the stimulus generator, the controller may compile a "differentiation map" or a depth profile of the varicose veins.

In some embodiments, the controller may include functionality for allowing personalization of the temperature stimuli (either heat or cold) provided by the device. This may allow for the device to be adaptable based on individual user characteristics, such as sensitivity to temperature and skin color.

In some embodiments, an intensity of the temperature stimuli (either the heating or cooling effect) can be adjusted to suit preferences of an individual in terms of the pain threshold or comfort level. Individuals differ in terms of their levels of sensitivity to temperature changes. A setting of the thermal stimulus generator may be configured to limit the heat or cold source, preventing discomfort or even pain.

In some embodiments, if the thermal stimulus is a cooling stimulus, the stimulus can provide a secondary function as a pain reduction element. Here, the cooling effect is serving two purposes. First, as a stimulus to measure body fluid flow changes. Second, as a pain relief element - by reducing discomfort or pain in the area where the device is applied.

In some embodiments, where the thermal stimulus is an optical thermal stimulus, the optical thermal stimuli may be adapted to the pigmentation of the user's skin.

Here, it is proposed to implement adaptation of the thermal stimuli based on the individual's skin color. Different skin colors can absorb and reflect light differently. This difference in light absorption can affect how much of an applied optical stimulus is converted into heat. Thus, personalizing the intensity of the optical stimulation to match an individual's skin pigmentation and maximum tolerable heat temperature can help prevent overheating the skin and causing discomfort or potential damage.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A device (30) for application in use to a tissue surface area (62), comprising:
a thermal stimulus generator (42) for applying a thermal stimulus (46) to one or more locations of the tissue on or beneath the tissue surface area;
a thermal imaging subsystem (52) including at least one thermal imaging camera (54), for thermally imaging a region of the tissue surface area;
a controller (32) adapted to:
receive thermal imaging data (44) acquired by the thermal imaging subsystem during or subsequent to application of the thermal stimulus, and
extract a thermal signal (72) from the thermal imaging data; and
determine body fluid flow information based on the extracted thermal signal.

2. The device of claim 1, wherein the thermal stimulus generator is adapted to generate one or more thermal stimulus pulses.

3. The device of claim 1 or 2, wherein the extracted thermal signal comprises a thermal pulse wave signal (72), and preferably wherein extracting the thermal signal comprises extracting a thermal pulse wave having a peak or center point which is displaced from a location of application of the thermal stimulus.

4. The device of claim 3, wherein the extracting the thermal signal comprises detecting in the thermal imaging data a thermal pulse wave of a pre-defined shape or mathematical form, for example a Gaussian pulse wave.

5. The device of any preceding claim, wherein the thermal imaging data comprises a time series of thermal image frames spanning an imaging period.

6. The device of claim 5,
wherein the extracting the thermal signal comprises detecting presence and/or location of a thermal pulse wave in each of at least a subset of the time series of thermal image frames to thereby obtain a thermal pulse wave signal comprising a sample of the thermal pulse wave at each of a series of sampling time points; and
optionally wherein the controller is adapted to determine at least one property of a travel path of the thermal pulse wave signal across an imaging field of view of the thermal imaging subsystem based on plotting a location of a fiducial point of the thermal pulse wave at each of a series of sampling time points.

7. The device of any of claims 3-6, wherein the thermal imaging data comprises a time series of thermal image frames spanning an imaging period, and wherein the controller is adapted to identify a temporally first frame of the series of frames in which the thermal pulse wave is present/detectable.

8. The device of any preceding claim,
wherein the controller is adapted to record extracted thermal signal data and/or body fluid flow information from multiple measurement sessions in a memory and is adapted to determine longitudinal body fluid flow information based thereon; and
optionally wherein the longitudinal body fluid flow information comprises an indication of a longitudinal trend in a value of a body fluid flow parameter over time and wherein the controller is adapted to apply a pre-defined diagnostic algorithm to the longitudinal trend, the diagnostic algorithm adapted to generate a diagnostic classification based on the input longitudinal blood flow trend.

9. The device of any preceding claim,
wherein the device comprises a tissue-facing area,
wherein the thermal stimulus generator and at least one thermal imaging camera of the thermal imaging subsystem are supported spaced from one another at the tissue facing area, for interaction with the tissue in use at tissue locations spaced from one another; and
optionally wherein the at least one thermal imaging camera is spaced from the thermal stimulus generator by a distance, x, and wherein a body fluid flow parameter is determined based on: a time delay between generation of a thermal stimulus by the thermal stimulus generator, and first detection of a resulting thermal pulse wave signal in an imaging field of view of the at least one thermal imaging camera.

10. The device of any preceding claim,
wherein the device is arranged such that, when applied in use to the tissue surface area, the thermal imaging subsystem is arranged for acquiring first thermal imaging data of at least a first tissue region and second thermal imaging data of a second tissue region, the first tissue region being spaced from the thermal stimulus generator in a first direction across the tissue surface and the second tissue region spaced from the thermal stimulus generator in a second direction across the tissue surface, opposite to the first direction; and
optionally wherein the thermal imaging subsystem comprises at least a first thermal imaging camera for acquiring the first thermal imaging data and a second thermal imaging camera for acquiring the second thermal imaging data, and wherein the first thermal imaging camera is spaced from the thermal stimulus generator in the first direction across the tissue-facing area of the device, and the second thermal imaging camera is spaced from the thermal stimulus in an opposite direction across a tissue facing area of the device.

11. The device of claim 10, wherein the controller is adapted to:
detect a thermal pulse wave signal in one of the first and second thermal imaging data;
measure a background thermal diffusion signal in the other of the first and second thermal imaging data;
estimate a background thermal diffusion signal present in the one of the first and second thermal imaging data having the thermal pulse wave signal based on the measured background thermal diffusion signal and subtract the estimated background diffusion signal from the detected thermal pulse wave signal to obtain a corrected thermal pulse wave signal;
determine the body fluid flow information based on the corrected thermal pulse wave signal.

12. The device of any of claims 1-11, wherein the controller is adapted to implement a sequential measurement process, for application of a thermal stimulus with the device positioned at a first location on the tissue, and acquisition of thermal imaging with the device positioned at a second location on the tissue, spaced from the first location, and the measurement process comprising:
controlling activation of the thermal stimulus;
controlling deactivation of the thermal stimulus;
controlling acquisition of the thermal imaging data subsequent to deactivation of the thermal stimulus, optionally after a pre-defined pause period.

13. The device of any of claims 1-12, wherein the body fluid is blood, and wherein the body fluid flow information is blood flow information.

14. The device of any preceding claim,
wherein the device is a personal care device, and
optionally wherein the personal care device is any one or more of:
an optical hair removal device, e.g. an IPL epilator device;
a shaver device;
a breast pump;
an oral healthcare device, such as an oral cleaning device, e.g. a toothbrush.

15. A method for detecting body fluid flow information, comprising:
applying a thermal stimulus to one or more locations of a tissue surface area;
using a thermal imaging subsystem including at least one thermal imaging camera to thermally image a region of the tissue surface area during or subsequent to application of the thermal stimulus;
extracting a thermal signal from the thermal imaging data; and
determining body fluid flow information based on the extracted thermal signal.
